# EUROPEAN PATENT SPECIFICATION

(11) **EP 3 630 010 B1**
(45) Date of publication and mention of the grant of the patent: **26.06.2024**
(21) Application number: 18770143.8
(22) Date of filing: 24.05.2018
(51) Int. Cl.: A61F 2/07, A61F 2/954, A61F 2/966

(54) **BRANCHED ENDOPROSTHESIS WITH TAIL FOR CONTROLLED BRANCH DEPLOYMENT**
VERZWEIGTE ENDOPROTHESE MIT TAIL ZUM KONTROLLIERTEN VERZWEIGUNGSEINSATZ
ENDOPROTHÈSE RAMIFIÉE À QUEUE POUR DÉPLOIEMENT DE RAMIFICATION COMMANDÉ

(30) Priority: 31.05.2017 US 201762512926 P
(43) Date of publication of application: 08.04.2020
(60) Divisional application: 24175814.3
(73) Proprietor: W. L. Gore & Associates, Inc., Newark, DE 19711 (US)
(72) Inventor: CHUNG, Karl R., Newark, Delaware 19711 (US); SECTOR, Martin J., Newark, Delaware 19711 (US); BEARD, Matthew S., Newark, Delaware 19711 (US)
(74) Representative: HGF
(86) International application number: PCT/US2018/034399
(87) International publication number: WO 2018/222494

(56) References cited:
- WO-A2-2014/096811
- US-A1- 2007 050 015

## Description

### FIELD

The present disclosure relates to branched endoprostheses for treating disease of the vasculature.

### BACKGROUND

Branched endoprostheses are commonly used for treating disease of the vasculature. By way of example, branched stent grafts may be used in the treatment of abdominal aortic aneurisms, which generally affect the abdominal aorta and may extend down into the iliac arteries.

A branched endoprosthesis used in the treatment of abdominal aortic aneurisms is generally inserted through an iliac artery up into the abdominal aorta, where it is deployed and anchored. A tubular ipsilateral leg may extend down into the iliac artery through which the graft was inserted, while a tubular contralateral leg may not extend below the abdominal aorta.

To extend the tubular contralateral leg down into the other iliac artery, a second endoprosthesis is inserted through that other iliac artery over a guidewire and attached to the original graft's tubular contralateral leg. Although endoscopic imaging, including radiopaque markers, may be employed, this cannulation process is often difficult given not only the tortuous vasculature, but also from structural biases within the original branched endoprosthesis angling the tubular legs apart in a Y configuration so as to face them toward their respective iliac arteries.

U.S. Application No. 13/740,457, filed January 14, 2013, and published as U.S. 2013/0211501 is directed to branched endoprostheses comprising graft and support components. The branched endoprostheses can comprise a body portion and legs, where the legs are maintained in an aligned configuration for ease of cannulation of at least one of the legs. WO2014/096811 is directed to a prosthesis comprising a graft sleeve having a first and second ends with a lumen therethrough, a main body portion bifurcated at a junction to form a first leg and a second leg such that the lumen of the graft sleeve in main body portion is divided into two smaller lumens. The body portion has two ring stents, first leg and second leg each have ring stents which maintain the patency of legs and assist bending of the leg graft without kinking to accommodate the patient's anatomy. The first leg and second leg each include a fenestration in the fabric material forming each leg. Corrugations in first leg and/or second leg allow a degree of twisting to accommodate angular adjustment of the fenestration position.

### SUMMARY

The invention is an endoprosthesis delivery system as defined by claim 1. This disclosure is directed to techniques for controlling branches of a branched endoprosthesis during deployment. The disclosed techniques include a tail extending from a branch following release of the branch from a constraint. The tail may remain constrained even after the branch assumes an expanded configuration. Thus, the tail may help orient the branch following release of the branch from the constraint.

In one example, this disclosure is directed to an endoprosthesis delivery system comprising a branched endoprosthesis having a long leg and a short leg, each leg having an end, the short leg including a tail that extends beyond the end of the short leg, a constraint attached to at least a portion of the long leg prior to deployment. The constraint also retains at least a portion of the tail. The constraint is configured to release both the long leg and the tail when the branched endoprosthesis is fully deployed.

In another example, this disclosure is directed to an endoprosthesis comprising a tubular body portion, a long leg with a distal end connected to an end of the tubular body portion, and an end extending away from the tubular body portion, a short leg with a distal end connected to the end of the tubular body portion, and an end extending away from the tubular body portion. The tubular body portion, the long leg and the short leg are formed from a graft component, and at least one support component. The endoprosthesis further comprises a tail extending from the end of the short leg, the tail being unsupported by the at least one support component. The tail is configured to be tucked under a constraint with undeployed sections of the long leg during deployment of the endoprosthesis.

In a further example, this disclosure is directed to a method comprising delivering an assembly comprising a branched endoprosthesis and constraint releasably retaining the branched endoprosthesis in a collapsed configuration to a trunk vessel. The branched endoprosthesis includes a tubular body portion, a long leg and a short leg, each leg having an end, the short leg including a tail that extends beyond the end of the short leg. The method further includes partially releasing the constraint such that a distal section of the branched endoprosthesis, the distal section including the tubular body portion, a distal portion of the long leg, and the short leg, assume an expanded configuration, with undeployed sections of the long leg and at least a portion of the tail remaining constrained by the constraint. The method further includes fully releasing the constraint to deploy the undeployed sections of the long leg and at least the portion of the tail.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 illustrates a branched stent graft including a tail for controlled branch deployment, according to some embodiments;
FIGS. 2A - 2C illustrate the progressive deployment of a branched stent graft with a tail, according to some embodiments;
FIG. 3 is a flowchart illustrating techniques for deploying a branched stent graft within a branched vasculature, according to some embodiments; and
FIGS. 4A - 4D illustrate tail configurations suitable for used with the branched stent graft of FIG 1, according to some embodiments.
FIGS. 5A and 5B illustrate the progressive deployment of a branched stent graft, with a cantilever configured to maintain alignment of a released branch, according to some embodiments;

Various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure. In that regard, the drawing figures should not be construed as limiting.

### DETAILED DESCRIPTION

Although various endoluminal delivery techniques are described with respect to stent grafts for repair of aneurysms, the disclosed techniques may be readily adapted to any branched endoprosthesis configured for deployment within a vasculature. For example, the disclosed techniques may be applied to treatment of vasculature occlusions. In addition, the present disclosure is described primarily with reference to treating disease of the abdominal aorta, such as aortic aneurysms or common iliac occlusions; however, the disclosure and principles may be applied to other disease of the vasculature or other body lumens, including, for example, any disease where a larger body lumen and one or more branch lumens are to be treated. Likewise, although the disclosure is described primarily with reference to bifurcated endoprostheses, it should be understood that the techniques of the disclosure may be applied to endoprostheses including any number of branches, for example, two, three, four or more.

The present disclosure is directed toward a branched endoprosthesis, such as branched stent graft 140. An endoprosthesis may comprise a graft component and at least one support component, such as in a stent graft.

A graft component, such as graft component 142, is generally any abluminal (i.e., outer, vessel surface) or luminal (i.e., inner, blood flow surface) covering configured to partially or substantially cover one or more support components.

In various embodiments, a graft component, such as graft component 142, comprises ePTFE. However, other useful materials for the graft component may comprise one or more of nylons, polycarbonates, polyethylenes, polypropylenes, polytetrafluoroethylenes, polyvinyl chlorides, polyurethanes, polysiloxanes, and other biocompatible materials.

A graft component, such as graft component 142, is fixedly secured or otherwise coupled at a single or a plurality of locations to the abluminal or luminal surface of the support component, for example, using one or more of taping, heat shrinking, adhesion and other processes known in the art. In some embodiments, a plurality of graft components are used and may be coupled to both the abluminal and luminal surfaces of the support component(s). In other embodiments, a plurality of graft components "sandwich" the support component(s), the graft components being attached to each other within voids of the support components.

In various embodiments, a support component, such as stent component 144, has dimensions appropriate for the given treatment and may provide structural support for the graft component of the endoluminal device and/or the vasculature to be treated. A support component, such as stent component 144, may be a stent comprised either of a wire including a helical configuration or be comprised of one or a plurality of rings. Among other configurations, the wire or a ring itself may be linear or have a sinusoidal or zig-zag pattern. Still another support component, such as stent component 144, may be cut from a tube and have any pattern suitable for the treatment.

The support component, such as stent component 144, can be comprised of a shape-memory material, such as nitinol. In other embodiments, however, the support component may be comprised of other materials, self-expandable or otherwise expandable (e.g., with a conventional balloon catheter or spring mechanism), such as various metals (e.g., stainless steel), alloys and polymers.

In various embodiments, the branched endoprosthesis, such as branched stent graft 140, comprises a tubular body portion 150 and at least two tubular legs 152, 154, which may be defined by graft component 142 and/or support component 144. The cross-section of tubular body portion 150 may be circular, ovoidal, or have polygonal features with or without curved features. The cross-sectional shape of the tubular body portion 150 may be either substantially constant or variable along its axial length. In like manner, the cross-sectional surface area of the tubular body portion 150 may be either substantially constant or variable along its axial length. In an embodiment of a bifurcated endoprosthesis, a cross-section of the tubular body portion 150 is substantially circular at its distal end but tapers to have an ovoidal rectangular cross-section with a smaller cross-sectional surface area in its bifurcation region adjacent tubular legs 152, 154.

In various embodiments, at least two tubular legs, such as tubular legs 152, 154, are in an aligned configuration for ease of cannulation. The alignment may be temporary until after guidewire insertion or cannulation. In various embodiments, aligning a plurality of tubular legs requires overcoming the aforementioned structural bias and/or unpredictable bending due to interactions with anatomical structures or bodily fluids, such as blood during deployment. As used herein, "align" or "aligned" means aligned axially, drawn together, parallel, and/or the state of the plane of a first leg being perpendicular to the axis of a second leg to which the first leg is aligned.

A "constraint," such as outer sheath 110, may be comprised of one or more of nylons, polycarbonates, polyethylenes, polypropylenes, polytetrafluoroethylenes, polyvinyl chlorides, polyurethanes, polysiloxanes, stainless steels, or other biocompatible materials. A constraint can be a sleeve or an introducer sheath. In yet other embodiments, a constraint is a tubular element, as that term has been defined herein.

The term "tubular element" includes any longitudinally extending structure with or without a lumen therethrough, for example a catheter. Thus, tubular elements include, but are not limited to, tubes with lumens, solid rods, hollow or solid wires (e.g., guidewires), hollow or solid stylets, metal tubes (e.g., hypotubes), polymer tubes, pull cords or tethers, fibers, filaments, electrical conductors, radiopaque elements, radioactive elements and radiographic elements. Tubular elements can be of any material and can have any cross-sectional shape including but not limited to profiles that are circular, oval, triangular, square, polygon shaped or randomly shaped.

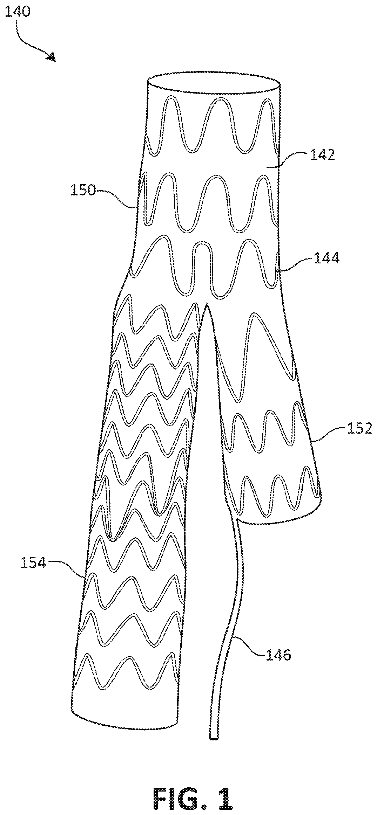

FIG. 1 illustrates a branched stent graft 140 including a graft component 142 and a support component 144. Stent graft 140 is a branched endoprosthesis including a tubular body portion 150, a tubular contralateral leg 152, and a tubular ipsilateral leg 154. Branched stent graft 140 provides a collapsed configuration for endoluminal delivery and an expanded configuration larger than collapsed configuration.

Tubular contralateral leg 152, and tubular ipsilateral leg 154 are branched off of and in luminal communication with the tubular body portion 150. Tubular ipsilateral leg 154 includes a distal end connected to an end of tubular body portion 150, and an end extending away from the tubular body portion. Likewise, tubular contralateral leg 152 includes a distal end connected to an end of tubular body portion 150, and an end extending away from the tubular body portion. Tubular contralateral leg 152 and tubular ipsilateral leg 154 join tubular body portion 150 at a bifurcated region of stent graft 140.

Tubular ipsilateral leg 154 and tubular contralateral leg 152 may be structurally biased to angle apart in a Y configuration, so as to face or direct them toward their respective vessels to be treated. The structural bias may arise from either or both of graft component 142 and support component 144.

In various embodiments, the axial length of tubular ipsilateral leg 154 is substantially longer that the axial length of tubular contralateral leg 152 such that end of tubular ipsilateral leg 154 is located proximal to end of tubular contralateral leg 152.

Graft component 142 includes a tail 146 extending from the end of tubular contralateral leg 152. Tail 146 is unsupported by support component 144 or any other support component. In some examples, tail 146 is seamless extension of a graft material forming graft component 142. For example, where graft component 142 forms a lumen at tubular contralateral leg 152, tail 146 may represent a partial section of the graft component 142 extending proximally beyond the end of the lumen. In other example, tail 146 may be a separate component connected to either graft component 142 or support component 144.

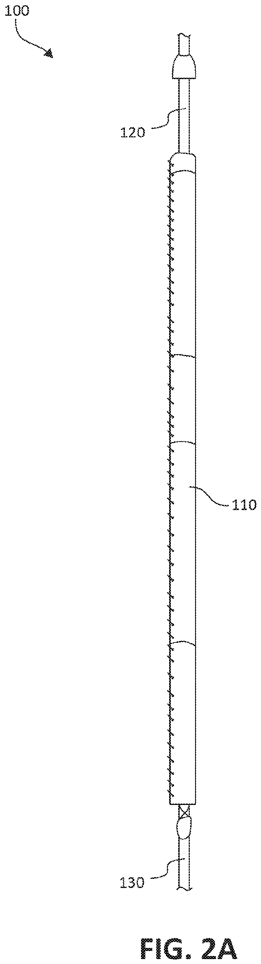

Tail 146 facilitates control of tubular contralateral leg 152 following release, and, in some examples, expansion of tubular contralateral leg 152 from a constraint such as a sheath or outer sleeve 110 (FIGS. 2A - 2C). For example, tail is configured to be tucked under a constraint, such as outer sleeve 110, with undeployed sections of tubular ipsilateral leg 154 during deployment of branched stent graft 140. Tail 146 is configured to maintain tubular contralateral leg 152, and tubular ipsilateral leg 154 in an aligned configuration during deployment of branched stent graft 140, e.g., to facilitate cannulation of tubular contralateral leg 152.

In various embodiments, tail 146 can comprise a thread, fiber, or filament, for example, one that is polymeric in nature. In other embodiments, tail 146 comprises a wire, including a high columnar strength. In yet other embodiments, tail 146 may be a tubular element, e.g., where the contralateral gate at the end of tubular contralateral leg 152 is provided by a cut in graft material of tubular contralateral leg 152. In some examples, the tail 146 is an elongate film member integrally formed with the contralateral leg 152. For example, the tail 146 may be formed from the same material as the graft component 142 (e.g., as part of a tape wrapping process), or otherwise bonded or secured to the first leg in an integral manner using any of the methods known to those of skill. In alternative examples, tail 146 may be integral with support component 144 or a combination of support component 144 and the graft component 142.

A length of tail 146 as measured extending away from tubular body portion 150 is greater than twice a cross-sectional width (e.g., the diameter) of end of tubular contralateral leg 152 as measured perpendicular to length of tail 146. In some embodiments, the end of tail 146 may be distally located relative to the end of tubular ipsilateral leg 154. In other embodiments, a combined length of tail 146 and tubular contralateral leg 152 is about equal to a length of tubular ipsilateral leg 154.

While specific dimensions of stent graft 140 are not germane to the inventions of this disclosure, dimensions suitable for treatment of some abdominal aortic aneurysms may be useful to provide context to this particular example of this disclosure. For example, for infrarenal aortic neck treatment a diameter range of 16-32 millimeters (mm) for stent graft 140 with a minimum aortic neck length of 10 mm provides suitable results.

In the same or different examples, stent graft 140 may provide proximal aortic neck angulation of less than or equal to 90 degrees, although a variety of angulations are contemplated.

In the same or different examples, stent graft 140 may provide an iliac treatment diameter range of 6.0 - 15 mm and iliac seal zone length of at least 10 mm, although a variety of dimensions are contemplated.

Similarly, in the treatment of common iliac aneurysms stent graft 140 may interface with one or both trunk gates. Stent graft 140 may provide a treatable of at least 124 mm from the lowest renal artery to the internal iliac artery, although a variety of dimensions are contemplated.

In the same or different examples, stent graft 140 may provide Iliac vessel diameters of 6.0 - 15 mm, and iliac seal zone length of at least 10 mm, although a variety of dimensions are contemplated.

FIGS. 2A - 2C illustrate the progressive deployment of branched stent graft 140 in an assembly 100 further including a guidewire 120 and a tubular element 130. Specifically, FIG. 2A illustrates an outer sheath 110 enclosing branched stent graft 140 (not shown) in a collapsed configuration for endoluminal delivery to be delivered via guidewire 120 and tubular element 130. In the collapsed configuration of FIG. 2A, tail 146 extends proximally from end of tubular contralateral leg 152 within outer sheath 110.

Outer sheath 110 extends around and maintains branched stent graft 140 in a delivery configuration. In such embodiments, outer sheath 110 can have opposite sides releasably held together by elongated member 112 to maintain branched stent graft 140 in the delivery configuration. In such embodiments, outer sheath 110 can have a plurality of holes through which elongated member 112 extends to releasably hold the opposite sides of outer sheath 110 together. In other examples, outer sheath 110 may be a tubular element that is torn or cut to release branched stent graft 140. In further examples, outer sheath 110 may be a tubular element that is retracted to release branched stent graft 140.

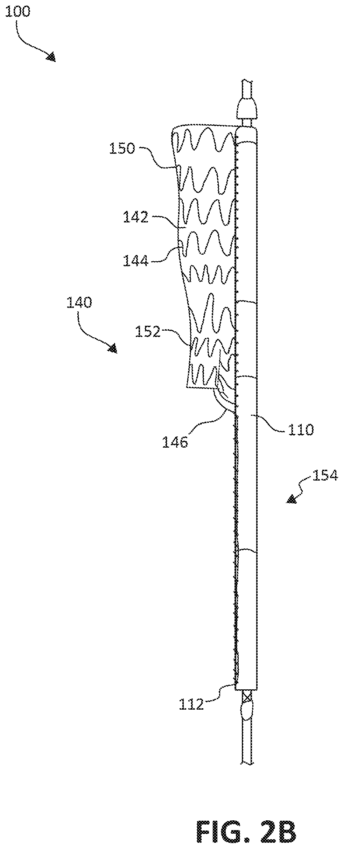

Turning now to FIG. 2B, outer sheath 110 is partially removed from the distal half of branched stent graft 140, revealing tail 146 extending from the end of tubular contralateral leg 152. For example, outer sheath 110 may form a luminal element with opposing sides held together via a removable elongated member 112. In various embodiments, elongated member 112 can comprise a thread, fiber, or filament, for example, one that is polymeric in nature.

Outer sheath 110, also described as a constraint, is configured to, during deployment of branched stent graft 140 from collapsed configuration to expanded configuration, first release a distal section of branched stent graft 140, distal section including tubular body portion 150, a distal portion of tubular ipsilateral leg 154, and tubular contralateral leg 152, with undeployed sections of tubular ipsilateral leg 154 and at least a portion of tail 146 remaining temporarily constrained by outer sheath 110. In different terms, the outer sheath is configured to secure the tail 146 adjacent to the ipsilateral leg 154 when the contralateral leg 152 is in the deployed state, such that the contralateral leg 152 is maintained in an alignment with the ipsilateral leg 154 when the contralateral leg 152 is in the deployed state.

Following the partial removal of outer sheath 110, i.e., removal of the distal portion of outer sheath, tubular contralateral leg 152 has assumed an expanded configuration larger than collapsed configuration. Tail 146 holds contralateral leg 152 in alignment with tubular ipsilateral leg 154 by preventing contralateral leg 152 from bending away from outer sheath 110. In this manner, when tucked under outer sheath 110 with undeployed sections of tubular ipsilateral leg 154, tail 146 maintains alignment of tubular ipsilateral leg 154 and tubular contralateral leg 152 during expansion of branched stent graft 140 from collapsed configuration to assist in cannulation of tubular contralateral leg 152.

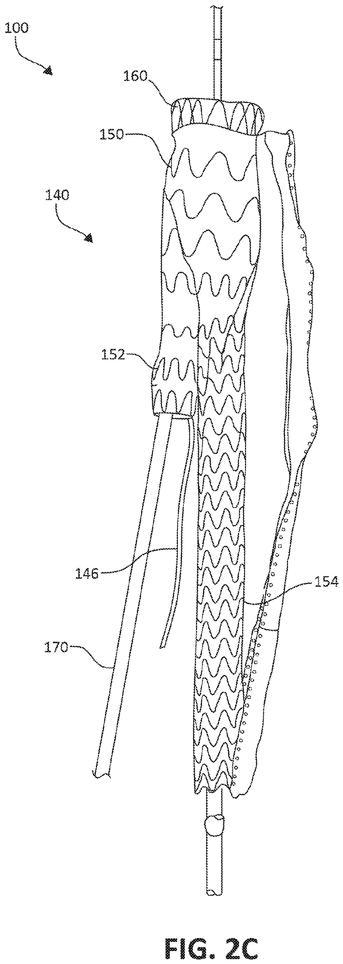

As shown in FIG. 2B, outer sheath 110 may be partially removed from the distal portion of branched stent graft 140, but not removed at its end, thus maintaining contralateral and tubular ipsilateral legs 152,154 of branched stent graft 140 in an aligned configuration until, as shown in FIG. 2C, a guidewire 170 has been inserted into tubular contralateral leg 152 of branched stent graft 140 or cannulation of tubular contralateral leg 152 has occurred. In various embodiments, alignment of contralateral and tubular ipsilateral legs 152,154 by tail 146 may provide a very minimal impact on, the crossing profile of assembly 100, for example to less than 18Fr, less than 16Fr, or less than 14Fr, although a variety of dimensions are contemplated.

With reference to FIG. 2C, anchors 160 at the distal end of branched stent graft 140 may be retracted for adjusting placement of branched stent graft 140. Note that in this embodiment, tail 146 still maintains the contralateral and tubular ipsilateral legs of branched stent graft 140 in an aligned configuration during the adjustment. Once released from outer sheath 110, branched stent graft 140 provides an expanded configuration larger than collapsed configuration.

Additional features and elements may be used in connection with the present disclosure. In one embodiment for example, tubular contralateral leg 152 is maintained in an open configuration for ease of cannulation. This may be accomplished, for example, by incorporating an independent wire or ring, such as a support component as described herein, at the distal end of contralateral leg 152. In some embodiments, a plurality of serially aligned support components are adapted to hold tubular contralateral leg 152 open for cannulation. In some embodiments, one or more radiopaque and/or echogenic markers are incorporated into the branched endoprosthesis, for example, along, or at, the end of tubular contralateral leg 152.

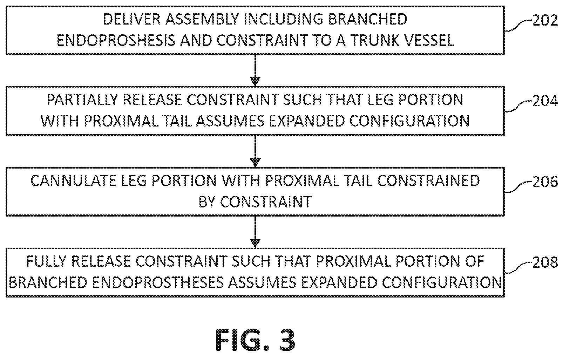

FIG. 3 is a flowchart illustrating techniques for deploying a branched stent graft within a branched vasculature. For clarity, the techniques of FIG. 3 are described with respect to assembly 100, including outer sheath 110 and branched stent graft 140.

First, assembly 100 is delivered in a collapsed configuration to a trunk vessel, such as an abdominal aorta (202). For example, branched stent graft 140 may be delivered enclosed by outer sheath 110 as part of assembly 100 into a branch vessel, such as a ipsilateral branch vessel, and to the lumen of a trunk vessel via guidewire 120 and a tubular element 130, for example at a distal end of a catheter.

Next, placement of branched stent graft 140 may be adjusted, for example, by retracting anchors 160 at the distal end of the tubular body portion 150 of the branched stent graft 140, rotating and/or advancing or reversing guidewire 120 and/or tubular element 130, and thereafter fully deploying anchors 160 into the sides of the trunk artery.

As shown in FIG. 2B, outer sheath 110 may be partially released, e.g., by partial removal of elongated member 112 (204). With the partial removal of elongated member 112 a distal section of branched stent graft 140, distal section including tubular body portion 150, a distal portion of tubular ipsilateral leg 154, and tubular contralateral leg 152, assume an expanded configuration. With outer sheath 110 partially released, undeployed sections of tubular ipsilateral leg 154 and at least a portion of tail 146 remain constrained by outer sheath 110.

For example, tubular ipsilateral leg 154 and tubular contralateral leg 152 may be biased to be angled apart in a Y configuration. With tail 146 constrained by outer sheath 110, the bias is temporarily overcome to align tubular ipsilateral leg 154 and tubular contralateral leg 152. Using the alignment of tubular ipsilateral leg 154 and tubular contralateral leg 152, the contralateral gate of contralateral leg 152 may be located to facilitate its connection to a contralateral vessel.

For example, tubular contralateral leg 152 may be cannulated (206). Such a cannulation method may include inserting a second guidewire into tubular contralateral leg 152 via a second branch artery in communication with the trunk artery. Cannulation of tubular contralateral leg 152 portion may thereafter occur.

Once the second guidewire has been inserted into tubular contralateral leg 152, outer sheath 110 may be fully removed, thereby releasing tail 146 (208). Branched stent graft 140 is allowed to assume an expanded configuration larger than the collapsed configuration. In addition, with tail 146 released, tail 146 no longer maintains tubular contralateral and ipsilateral legs 152,154 of the branched stent graft 140 in an aligned configuration. Finally, tubular ipsilateral leg 154 and tubular contralateral leg 152 are allowed to return to the initial Y configuration.

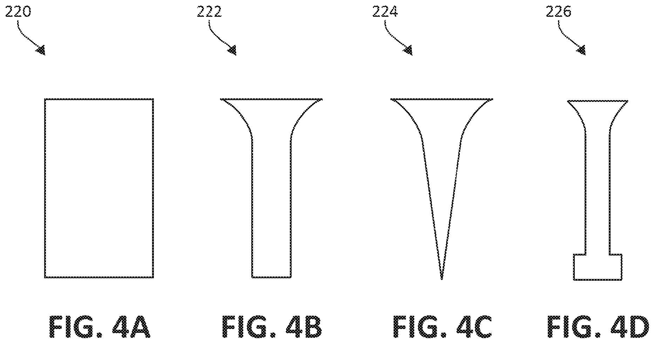

FIGS. 4A - 4D illustrate example tail configurations for tail 146 of branched stent graft 140. The examples of FIGS. 4A - 4D are not exhaustive any number of other profiles may be utilized for tail 146 of branched stent graft 140.

As shown in FIG. 4A, tail 220 is rectangular. As an example, tail 220 may be as wide as diameter of tubular contralateral leg 152. In a specific example, tail 220 is 18 mm wide, although a variety of dimensions are contemplated.

As shown in FIG. 4B, tail 222 is tapered. As compared to tail 220, tail 222 provides a reduced geometry featuring a strain relief from the contralateral gate of tubular contralateral leg 152 to mitigate tearing of graft material 142. For example, tail 222 may taper to 2 - 7 mm wide, such as about 4 mm wide or about 6 mm wide, although a variety of dimensions are contemplated.

As shown in FIG. 4C, tail 224 is tapered. As compared to tail 222, tail 224 provides a pointed proximal tip. Like tail 222, tail 224 features a strain relief from the contralateral gate of tubular contralateral leg 152 to mitigate tearing of graft material 142. For example, tail 224 may have an initial diameter of 2 - 7 mm wide, such as about 3 mm wide or about 5 mm wide, although a variety of dimensions are contemplated.

As shown in FIG. 4D, tail 226 rectangular with an enlarged tip providing a modified dog bone shape. As compared to tail 220, tail 226 limits surface area and packing density in the central portion of the extended trim while increasing retention force due to the flared bottom of the trim. For example, tail 226 may have an initial diameter of 2 - 7 mm wide, and a wider expanded tip. In one particular example, the initial diameter is about 3 mm wide and the tip is about 5 mm wide, although a variety of dimensions are contemplated.

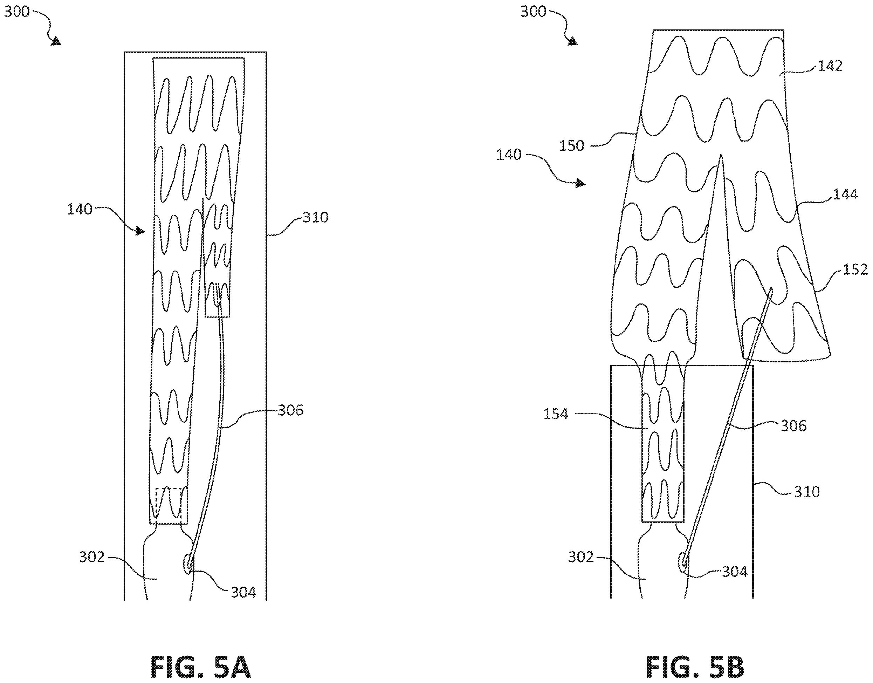

FIGS. 5A and 5B illustrate the progressive deployment of branched stent graft 140 in an assembly 300 further including an elongate element 306 and a tubular element 302. Specifically, FIG. 2A illustrates an outer sheath 310 enclosing branched stent graft 140 in a collapsed configuration for endoluminal delivery. In the collapsed configuration, elongate element 306 extends from lumen 304 of tubular element 302 and captures the proximal end of tubular contralateral leg 152.

Outer sheath 310 extends around and maintains branched stent graft 140 in a delivery configuration. Outer sheath 310 is a tubular element that is retracted to release branched stent graft 140, as shown in FIG. 5B. In other examples, outer sheath 310 may be a tubular element that is torn or cut to release branched stent graft 140. In further examples, outer sheath 310 can have opposite sides releasably held together by an elongated member (as with outer sheath 110) to maintain branched stent graft 140 in the delivery configuration.

Outer sheath 310, also described as a constraint, is configured to, during deployment of branched stent graft 140 from collapsed configuration to expanded configuration, first release a distal section of branched stent graft 140, distal section including tubular body portion 150, a distal portion of tubular ipsilateral leg 154, and tubular contralateral leg 152, with undeployed sections of tubular ipsilateral leg 154 remaining temporarily constrained by outer sheath 310.

Turning now to FIG. 5B, outer sheath 310 is partially removed from the distal half of branched stent graft 140, allowing the expansion of tubular body portion 150, a distal portion of tubular ipsilateral leg 154, and tubular contralateral leg 152. Elongate element 306 extends from lumen 304 of tubular element 302 and captures the proximal end of tubular contralateral leg 152, such that the tubular contralateral leg 152 is maintained in an alignment with the tubular ipsilateral leg 154 when the tubular contralateral leg 152 is in the deployed state.

Following the partial removal of outer sheath 310, i.e., removal of the distal portion of outer sheath, tubular contralateral leg 152 has assumed an expanded configuration larger than collapsed configuration. Elongate element 306 holds tubular contralateral leg 152 in alignment with tubular ipsilateral leg 154 by preventing tubular contralateral leg 152 from bending away from outer sheath 310. In various examples, elongate element 306 may represent a wire or hypotube or other elongate element suitable to extend from lumen 304 into the proximal end of tubular contralateral leg 152.

As shown in FIG. 5B, outer sheath 310 may be partially removed from the distal portion of branched stent graft 140, but not removed at its end, thus maintaining contralateral and tubular ipsilateral legs 152,154 of branched stent graft 140 in an aligned configuration until, elongate element 306 is retracted to release contralateral leg 152 (not shown).

Additional features and elements may be used in connection with the present disclosure. In one embodiment for example, contralateral leg 152 is maintained in an open configuration for ease of cannulation. This may be accomplished, for example, by incorporating an independent wire or ring, such as a support component as described herein, at the distal end of contralateral leg 152. In some embodiments, a plurality of serially aligned support components are adapted to hold contralateral leg 152 open for cannulation. In some embodiments, one or more radiopaque and/or echogenic markers are incorporated into the branched endoprosthesis, for example, along, or at, the end of contralateral leg 152. In various examples, elongate element 306 may be retracted once contralateral leg 152 is captured by a separate guidewire to facilitate cannulation.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims.

## Claims

1. An endoprosthesis delivery system comprising:
a branched endoprosthesis (140) having a long leg (154) and a short leg (152), each leg having an end,
the short leg (152) including a tail (146, 220, 222, 224, 226), the tail is a seamless extension of a graft material forming a graft component (142) of the branched endoprosthesis (140) that extends beyond the end of the short leg (152); and
a constraint (110) attached to at least a portion of the long leg (154) prior to deployment,
wherein the constraint (110) also retains at least a portion of the tail (146, 220, 222, 224, 226),
wherein the constraint (110) is configured to release both the long leg (154) and the tail (146, 220, 222, 224, 226) when the branched endoprosthesis (140) is fully deployed,
wherein when constrained by the constraint (110) with a distal portion of the long leg (154) during deployment of the branched endoprosthesis (140), the tail (146, 220, 222, 224, 226) is configured to couple together and maintain alignment of the long leg (154) and the short leg (152) to assist in cannulation of the short leg (152), and
wherein the constraint (110) is configured to, during deployment of the branched endoprosthesis (140) from a collapsed configuration to an expanded configuration, first release a distal section of the branched endoprosthesis (140), the distal section including a tubular body portion (150), the distal portion of the long leg (154), and the short leg (152), with undeployed sections of the long leg (154) and at least a portion of the tail (146, 220, 222, 224, 226) remaining temporarily constrained by the constraint (110).

2. The endoprosthesis delivery system of claim 1, wherein a combined length of the tail (146, 220, 222, 224, 226) and the short leg (152) is about equal to a length of the long leg (154).

3. The endoprosthesis delivery system of claim 1, wherein the long leg (154) and the short leg (152) are structurally biased to angle apart in a Y configuration.

4. The endoprosthesis delivery system of claim 1, wherein a length of the tail (146, 220, 222, 224, 226) as measured extending away from the second end of the short leg (152) is greater than twice a cross-sectional width of the second end of the short leg (152) as measured perpendicular to the length of the tail (146, 220, 222, 224, 226).

## Patentansprüche

1. Endoprothesen-Zuführsystem, umfassend:
eine verzweigte Endoprothese (140) mit einem langen Schenkel (154) und einem kurzen Schenkel (152), wobei jeder Schenkel ein Ende aufweist,
wobei der kurze Schenkel (152) einen Fortsatz (146, 220, 222, 224, 226) enthält, wobei der Fortsatz eine nahtlose Verlängerung eines Transplantatmaterials ist, das eine Transplantatkomponente (142) der verzweigten Endoprothese (140) bildet, die sich über das Ende des kurzen Schenkels (152) hinaus erstreckt; und
eine Begrenzung (110), die vor der Entfaltung an mindestens einem Abschnitt des langen Schenkels (154) angebracht ist,
wobei die Begrenzung (110) auch mindestens einen Abschnitt des Fortsatzes (146, 220, 222, 224, 226) festhält,
wobei die Begrenzung (110) dazu konfiguriert ist, sowohl den langen Schenkel (154) als auch den Fortsatz (146, 220, 222, 224, 226) freizugeben, wenn die verzweigte Endoprothese (140) vollständig entfaltet ist,
wobei, wenn er durch die Begrenzung (110) mit einem distalen Abschnitt des langen Schenkels (154) während der Entfaltung der verzweigten Endoprothese (140) begrenzt ist, der Fortsatz (146, 220, 222, 224, 226) dazu konfiguriert ist, den langen Schenkel (154) und den kurzen Schenkel (152) miteinander zu koppeln und ihre Ausrichtung zu erhalten, um die Kanülierung des kurzen Schenkels (152) zu unterstützen, und
wobei die Begrenzung (110) dazu konfiguriert ist, während der Entfaltung der verzweigten Endoprothese (140) von einer zusammengeklappten Konfiguration in eine ausgedehnte Konfiguration zuerst einen distalen Bereich der verzweigten Endoprothese (140) freizugeben, wobei der distale Bereich einen röhrenförmigen Körperabschnitt (150), den distalen Abschnitt des langen Schenkels (154) und den kurzen Schenkel (152) enthält, wobei nicht entfaltete Bereiche des langen Schenkels (154) und mindestens ein Abschnitt des Fortsatzes (146, 220, 222, 224, 226) vorübergehend durch die Begrenzung (110) begrenzt bleiben.

2. Endoprothesen-Zuführsystem nach Anspruch 1, wobei eine kombinierte Länge des Fortsatzes (146, 220, 222, 224, 226) und des kurzen Schenkels (152) etwa gleich einer Länge des langen Schenkels (154) ist.

3. Endoprothesen-Zuführsystem nach Anspruch 1, wobei der lange Schenkel (154) und der kurze Schenkel (152) strukturell so vorgespannt sind, dass sie in einer Y-Konfiguration voneinander abgewinkelt sind.

4. Endoprothesen-Zuführsystem nach Anspruch 1, wobei eine Länge des Fortsatzes (146, 220, 222, 224, 226), gemessen in der Erstreckung von dem zweiten Ende des kurzen Schenkels (152) weg, größer als die doppelte Querschnittsbreite des zweiten Endes des kurzen Schenkels (152), gemessen senkrecht zu der Länge des Fortsatzes (146, 220, 222, 224, 226), ist.

## Revendications

1. Système de pose d'endoprothèse comprenant :
une endoprothèse ramifiée (140) comportant une jambe longue (154) et une jambe courte (152), chaque jambe comportant une extrémité,
la jambe courte (152) comprenant une queue (146, 220, 222, 224, 226), la queue est une extension continue d'un matériau de greffon formant un composant de greffon (142) de l'endoprothèse ramifiée (140) qui s'étend au-delà de l'extrémité de la jambe courte (152) ; et
une contrainte (110) fixée à au moins une partie de la jambe longue (154) avant déploiement,
ladite contrainte (110) retenant également au moins une partie de la queue (146, 220, 222, 224, 226),
ladite contrainte (110) étant conçue pour libérer à la fois la jambe longue (154) et la queue (146, 220, 222, 224, 226) lorsque l'endoprothèse ramifiée (140) est entièrement déployée,
lorsqu'elle est contrainte par la contrainte (110) avec une partie distale de la jambe longue (154) durant le déploiement de l'endoprothèse ramifiée (140), la queue (146, 220, 222, 224, 226) étant conçue pour coupler ensemble la jambe longue (154) et la jambe courte (152) et maintenir l'alignement de celles-ci afin de faciliter la canulation de la jambe courte (152), et
ladite contrainte (110) étant conçue pour, durant le déploiement de l'endoprothèse ramifiée (140) d'une configuration repliée à une configuration déployée, libérer d'abord une section distale de l'endoprothèse ramifiée (140), la section distale comprenant une partie de corps tubulaire (150), la partie distale de la jambe longue (154) et la jambe courte (152), avec des sections non déployées de la jambe longue (154) et au moins une partie de la queue (146, 220, 222, 224, 226) qui restent temporairement contraintes par la contrainte (110).

2. Système de pose d'endoprothèse de la revendication 1, une longueur combinée de la queue (146, 220, 222, 224, 226) et de la jambe courte (152) étant environ égale à une longueur de la jambe longue (154).

3. Système de pose d'endoprothèse de la revendication 1, ladite jambe longue (154) et ladite jambe courte (152) étant structurellement sollicitées pour s'écarter selon un angle dans une configuration en Y.

4. Système de pose d'endoprothèse de la revendication 1, une longueur de la queue (146, 220, 222, 224, 226), telle que mesurée s'étendant à partir de la seconde extrémité de la jambe courte (152), étant supérieure à deux fois une largeur de coupe transversale de la seconde extrémité de la jambe courte (152) telle que mesurée perpendiculairement à la longueur de la queue (146, 220, 222, 224, 226).
